# EUROPÄISCHE PATENTANMELDUNG

(11) **EP 1 396 232 A1**
(43) Veröffentlichungstag der Anmeldung: **10.03.2004**
(21) Anmeldenummer: 03020269.1
(22) Anmeldetag: 08.09.2003
(51) Int. Cl.: A61B 17/32

(54) **Ultraschallinstrument**

(30) Priorität: 09.09.2002 DE 10241702
(71) Anmelder: Berchtold Holding GmbH, 78532 Tuttlingen (DE)
(72) Erfinder: Fritzsch, Gernod, Dr.-Ing., 78532 Tuttlingen (DE)
(74) Vertreter: Manitz, Finsterwald & Partner GbR

(57) **Zusammenfassung**

Ein chirurgisches Ultraschallinstrument weist ein Gehäuse mit einem Ultraschallwandler auf, wobei mit dem Gehäuse ein Schaft verbunden ist, an dessen distalem Ende eine Applikationsanordnung vorgesehen ist. Die Applikationsanordnung umfasst ein von dem Ultraschallwandler schwingend angetriebenes Arbeitselement und ein feststehendes Arbeitselement, zwischen denen ein Arbeitsraum zur Aufnahme von Gewebe gebildet ist.

## Beschreibung

Die vorliegende Erfindung betrifft ein chirurgisches Ultraschallinstrument mit einem Gehäuse, das einen Ultraschallwandler aufweist, sowie mit einem mit dem Gehäuse verbundenen Schaftabschnitt, an dessen distalem Ende eine Applikationsanordnung vorgesehen ist, wobei die Applikationsanordnung ein von dem Ultraschallwandler in Axialrichtung des Schaftes schwingend angetriebenes Arbeitselement und ein in Axialrichtung feststehendes Arbeitselement aufweist.

Chirurgische Ultraschallinstrumente dieser Art sind als so genannte Ultraschallscheren grundsätzlich bekannt und haben sich in den letzten Jahren zum Trennen und Präparieren von Gewebe bewährt. Ultraschallscheren werden zum Trennen und/oder Koagulieren von Gewebe in der endoskopischen und offenen Chirurgie genutzt.

Bei bekannten Ultraschallscheren wird eine gelenkig montierte Klammer gegen eine Klinge gedrückt, die durch einen Ultraschallwandler in Schwingungen versetzt wird und dadurch thermische Effekte im Gewebe auslöst. In Abhängigkeit von dem Druck auf das Gewebe und in Abhängigkeit von der vorgesehenen Applikationsfläche kann ein Schneiden oder Koagulieren erfolgen.

Bei den bekannten Ultraschallscheren besteht das Problem, dass die bewegliche Klemmbacke eine aufwändige Konstruktion erfordert, was den Preis des Instrumentes erhöht. Insbesondere endoskopische Instrumente mit relativ dünnem Schaft (beispielsweise 5 mm) sind sehr diffizil und mechanisch empfindlich. Darüber hinaus sind die bekannten Ultraschallscheren nur teilweise zerlegbar und schlecht reinigbar, was im Hinblick auf eine Wiederverwendung nachteilig ist.

Es ist die Aufgabe der vorliegenden Erfindung, ein chirurgisches Ultraschallinstrument zu schaffen, das einfach und kostengünstig herstellbar ist und das leicht gereinigt werden kann.

Diese Lösung dieser Aufgabe erfolgt durch die Merkmale des Anspruchs 1 und bei einem Instrument der eingangs genannten Art insbesondere dadurch, dass das feststehende Arbeitselement unbeweglich mit dem Schaft verbunden ist, und dass das feststehende und/oder das angetriebene Arbeitselement derart geformt sind, dass zwischen diesen ein Arbeitsraum gebildet ist, der sich in proximaler Richtung verjüngt.

Erfindungsgemäß ist somit am distalen Ende des Schaftes eine Applikationsanordnung vorgesehen, die einen sich in proximaler Richtung verengenden Spalt in Form einer gabelförmigen Öffnung aufweist, in die das zu behandelnde Gewebe hineingeschoben werden kann. Durch den sich bei Einschieben des Gewebes in die gabelförmige Öffnung stetig erhöhenden Druck wird das Gewebe in dem sich verjüngenden Spalt gegen das angetriebene, das heißt das in Axialrichtung des Instrumentes schwingende Arbeitselement, gedrückt, wobei das Gewebe koaguliert und/ oder durchtrennt wird. Hierbei werden unter den Arbeitselementen erfindungsgemäß diejenigen Elemente verstanden, die Gewebe zwischen sich aufnehmen und durch Zusammenwirken ein Schneiden bzw. Koagulieren bewirken.

Das erfindungsgemäße Instrument ist einfach im Aufbau, robust, leicht zerlegbar und leicht reinigbar. Es lässt sich wesentlich kostengünstiger als eine Ultraschallschere herstellen und für viele Indikationen nutzen.

Vorteilhafte Ausführungsformen der Erfindung sind in der Beschreibung, der Zeichnung, sowie den Unteransprüchen beschrieben.

Nach einer ersten vorteilhaften Ausführungsform kann das feststehende Arbeitselement Teil einer Schutzhülse sein. Hierdurch ist eine besonders einfache Bauweise und gleichzeitig eine gute Reinigungsmöglichkeit gegeben.

Nach einer weiteren vorteilhaften Ausführungsform tritt das bewegliche Arbeitselement aus einer in dem feststehenden Arbeitselement vorgesehenen Stirnöffnung aus. Auch hierdurch ergibt sich eine kompakte, schlanke Bauweise.

Nach einer weiteren Ausbildung der Erfindung kann das feststehende Arbeitselement eine zur Axialrichtung schräg verlaufende Stirnfläche aufweisen, die insbesondere insgesamt eben ausgebildet sein kann. Bei dieser Ausführungsform ergibt sich aufgrund der eben ausgebildeten Stirnfläche eine kostengünstige Herstellung. Ferner kann die Schneid- bzw. Koagulationswirkung durch die Gestaltung der Stirnfläche des feststehenden Arbeitselementes beeinflusst werden. Aus diesem Grund kann es vorteilhaft sein, die Stirnfläche des feststehenden Arbeitselementes zumindest teilweise gekrümmt auszubilden oder auf der Stirnfläche zur Längsachse des Instrumentes verschieden stark geneigte und/oder gekrümmte Abschnitte vorzusehen, um den Druck, mit dem das Gewebe gegen das bewegliche Arbeitselement gepresst wird, zu optimieren.

Nach einer weiteren Ausführungsform weist das feststehende Arbeitselement verglichen zu dem Schaftabschnitt seitliche Verjüngungen auf. Hierdurch wird die Sicht des Operateurs im Arbeitsbereich deutlich verbessert.

Nach einer weiteren Ausführungsform der Erfindung ist das bewegliche Arbeitselement rotationssymmetrisch ausgebildet. Hierbei kann es vorteilhaft sein, das bewegliche Arbeitselement sich in distaler Richtung konisch verjüngend auszubilden, da hierdurch die Höhe des Einlassspaltes, der die Arbeitshöhe darstellt, vergrößert wird.

Nach einer weiteren Ausbildung der Erfindung kann ein Arbeitselement relativ zu dem anderen um seine Längsachse verstellbar sein und mindestens zwei unterschiedlich gestaltete Arbeitsflächen aufweisen. Hierdurch ist es möglich, durch Drehen des einen Arbeitselementes relativ zu dem anderen unterschiedlich gestaltete Arbeitsflächen einzustellen. Beispielsweise kann eine scharfe Schneidkante oder aber eine Koagulationsfläche eingestellt werden, wodurch die Schneid- oder Koagulationseigenschaften bevorzugt gewählt werden können.

Nach einer weiteren vorteilhaften Ausführungsform der Erfindung können das bewegliche Arbeitselement und das feststehende Arbeitselement lösbar, und insbesondere mit wenigen Handgriffen leicht lösbar, miteinander verbunden sein. Hierdurch ergibt sich eine besonders leichte Reinigung des Instrumentes.

Nachfolgend wird die vorliegende Erfindung rein beispielhaft anhand vorteilhafter Ausführungsformen und unter Bezugnahme auf die beigefügten Zeichnungen beschrieben. Es zeigen:
- Fig. 1: eine schematische Seitenansicht einer ersten Ausführungsform eines chirurgischen Ultraschallinstrumentes;
- Fig. 2A: eine vergrößerte Ansicht des Bereichs II von Fig. 1 von unten;
- Fig. 2B: eine vergrößerte Seitenansicht des Bereichs II von Fig. 1;
- Fig. 3: eine Seitenansicht einer weiteren Ausführungsform entsprechend der Darstellung von Fig. 2B; und
- Fig. 4: eine Darstellung einer weiteren Ausführungsform entsprechend der Darstellung von Fig. 2B.

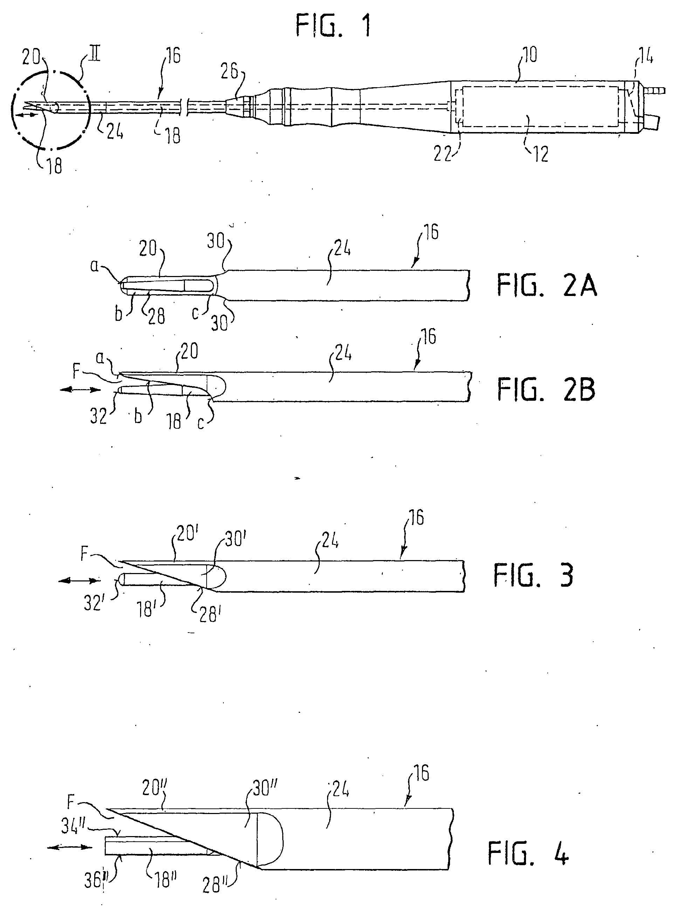

Das in Fig. 1 dargestellte chirurgische Ultraschallinstrument weist ein Gehäuse 10 auf, in dem ein Ultraschallwandler 12 vorgesehen ist, der über ein aus dem Gehäuse 10 herausführbares Kabel 14 mit elektrischer Energie versorgt werden kann. Das Gehäuse 10 ist im Wesentlichen zylindrisch ausgebildet und weist eine solche Größe auf, dass es vom Operateur mit der Hand leicht gegriffen werden kann. Alternativ kann das Gehäuse auch einen eigenen Handgriff und/oder Betätigungsschalter aufweisen.

Mit dem distalen Ende des Gehäuses 10 ist ein zylindrischer Schaft 16 verbunden, an dessen distalem Ende eine Applikationsanordnung vorgesehen ist, die ein angetriebenes Arbeitselement 18 und ein feststehendes Arbeitselement 20 aufweist, die jeweils einander zugewandte Arbeitsflächen aufweisen, und die in axialer Richtung etwa gleich lang sind, d.h. das eine Arbeitselement steht nicht über das andere Arbeitselement vor. Das angetriebene Arbeitselement 18 steht über ein Koppelelement 22 mit dem Ultraschallwandler 12 in Verbindung und wird von diesem in Schwingungen entlang der Längsachse des Instrumentes (vgl. Doppelpfeil in Fig. 1) versetzt. Hierbei ist das feststehende Arbeitselement 20 bei dem dargestellten Ausführungsbeispiel starr und unbeweglich mit dem Schaft 16 verbunden. Grundsätzlich kommt jedoch auch eine Ausführungsform in Betracht, bei der das feststehende Arbeitselement zwar unbeweglich mit dem Schaft verbunden, jedoch insgesamt flexibel ausgebildet ist. Bei der in Fig. 1 dargestellten Ausführungsform ist das feststehende Arbeitselement 20 Teil einer Schutzhülse 24, das heißt das feststehende Arbeitselement 20 ist einstückig mit der Schutzhülse 24 verbunden, die über eine Schraubmuffe 26 mit dem Gehäuse 10 verbunden ist.

Das bewegliche Arbeitselement 18 ist koaxial und innerhalb der Schutzhülse 24 geführt und tritt am distalen Ende des Instrumentes aus einer in dem feststehenden Arbeitselement 20 vorgesehenen Stirnöffnung aus (vgl. Fig. 2A).

Wie Fig. 2A und 2B zeigen, ist das bewegliche Arbeitselement 18 rotationssymmetrisch ausgebildet und weist eine kuppelförmig abgerundete Spitze 32 auf.

Wie Fig. 2 ferner zeigt, ist durch die schräg verlaufende Stirnfläche 28 des feststehenden Arbeitselementes 20 zwischen dieser Stirnfläche 28 und dem angetriebenen Arbeitselement 18 ein Arbeitsraum F gebildet, der sich in proximaler Richtung verjüngt. Mit anderen Worten ist zwischen dem feststehenden Arbeitselement 20 und dem angetriebenen Arbeitselement 18 eine gabelförmige Öffnung geschaffen, die einen sich stetig verjüngenden Spalt bildet.

Bei der in den Fig. 1 - 3 dargestellten Ausführungsform weist das feststehende Arbeitselement 20 eine Stirnfläche 28 auf, die mehrere verschieden stark geneigte und gekrümmte Abschnitte aufweist. So ist am vordersten distalen Ende des feststehenden Arbeitselementes 20 zunächst ein konvex gekrümmter Abschnitt a vorgesehen, an den sich ein linearer, das heißt ebener Abschnitt b anschließt, der schließlich in einen konkav gekrümmten Abschnitt c übergeht. Im Bereich der Stirnfläche 28 ist das feststehende Arbeitselement 20 - verglichen zu dem Schaft 24 - beidseitig seitlich verjüngt, so dass dort jeweils seitliche Verjüngungen 30 gebildet sind, welche das vordere Ende des Schaftes 24 in Draufsicht verschmälern.

Das bewegliche Arbeitselement 18 dieser Ausführungsform verjüngt sich in distaler Richtung konisch. Hierdurch ist die Arbeitshöhe des Arbeitsraums F vergrößert.

Fig. 3 zeigt entsprechend Fig. 2B eine Seitenansicht einer weiteren Ausführungsform eines chirurgischen Ultraschallinstrumentes, wobei im Übrigen die nicht dargestellten Bauteile der Ausführungsform von Fig. 1 entsprechen.

Wie Fig. 3 zeigt, weist bei dieser Ausführungsform das feststehende Arbeitselement 20' eine unter einem Winkel von etwa 20 bis 30° zur Längsachse des Instrumentes schräg verlaufende Stirnfläche 28' auf, die insgesamt eben ausgebildet ist. Ähnlich wie bei der Ausführungsform von Fig. 2 sind an dem feststehenden Arbeitselement 20' seitliche Verjüngungen 30' vorgesehen. Das bewegliche Arbeitselement 18' ist kreiszylindrisch ausgebildet und weist ebenfalls eine abgerundete Spitze 32' auf.

Fig. 4 zeigt eine weitere Ausführungsform eines chirurgischen Ultraschallinstrumentes, wobei die nicht dargestellten Bauelemente der Ausführungsform der Fig. 1 - 3 entsprechen. Bei dem in Fig. 4 dargestellten Instrument ist das feststehende Arbeitselement 20" ähnlich wie bei der Ausführungsform von Fig. 3 ausgebildet, das heißt es besitzt eine insgesamt eben ausgebildete Stirnfläche 28", aus der das bewegliche Arbeitselement 18" austritt. Ebenso ist das feststehende Arbeitselement 20" beidseitig mit Verjüngungen 30" versehen.

Das bewegliche Arbeitselement 18" ist um seine Längsachse verstellbar, das heißt es kann um seine Längsachse verdreht werden, wodurch unterschiedlich gestaltete Arbeitsflächen 34" und 36" einstellbar sind. Bei der in Fig. 4 dargestellten Position liegt eine schneidenförmige oder klingenförmige Arbeitsfläche 34" gegenüber der Stirnfläche 28", so dass bei Einführen von Gewebe in den Arbeitsraum F dieses durch die Keilwirkung des Arbeitsraums gegen die Arbeitsfläche 34" gedrückt wird, wodurch ein Schneiden des Gewebes erfolgt. Durch Drehen des angetriebenen Arbeitselementes 18" um seine Längsachse um 180° kann die weitere Arbeitsfläche 36" eingestellt werden, die flach bzw. eben ausgebildet ist, so dass bei Einführen von Gewebe in den Arbeitsraum F überwiegend eine Koagulationswirkung erzielt wird. Das Verdrehen des angetriebenen Arbeitselementes 18" um seine Längsachse kann mit Hilfe eines nicht dargestellten Handrades oder dergleichen erfolgen.

Bei Einsatz des oben beschriebenen Ultraschallinstrumentes wird der Ultraschallwandler 12 über die Leitung 14 mit elektrischer Energie versorgt, wodurch dieser das Koppelelement 22 und dadurch auch das angetriebene Arbeitselement 18 in Schwingungen versetzt, die parallel zur Längsachse des Instrumentes verlaufen. Durch Einführen von Gewebe in den Arbeitsraum F zwischen dem angetriebenen Arbeitselement und dem feststehenden Arbeitselement wird das Gewebe koaguliert und/oder durchtrennt.

Das feststehende Arbeitselement und das bewegliche Arbeitselement können jeweils aus unterschiedlichen Materialien bestehen, beispielsweise aus Titan und aus Kunststoff.

### Bezugszeichenliste

- 10: Gehäuse
- 12: Ultraschallwandler
- 14: Leitung
- 16: Schaft
- 18, 18', 18": angetriebenes Arbeitselement
- 20, 20', 20": feststehendes Arbeitselement
- 22: Koppelelement
- 24: Schutzhülse
- 26: Schraubmuffe
- 28, 28', 28": Stirnfläche
- 30, 30', 30": Verjüngung
- 32, 32': kuppelförmige Spitze
- 34": klingenförmige Arbeitsfläche
- 36": abgeflachte Arbeitsfläche
- a, b, c: Abschnitte der Stirnfläche
- F: Arbeitsraum

## Patentansprüche

1. Chirurgisches Ultraschallinstrument, mit
einem Gehäuse (10), das einen Ultraschallwandler (12) aufweist,
einem mit dem Gehäuse (10) verbundenen Schaft (16), an dessen distalem Ende eine Applikatiorisanordnung vorgesehen ist,
wobei die Applikationsanordnung ein von dem Ultraschallwandler (12) in Axialrichtung des Schaftes schwingend angetriebenes Arbeitselement (18, 18', 18") und ein in Axialrichtung feststehendes Arbeitselement (20, 20', 20") aufweist, wobei
das feststehende Arbeitselement (20, 20', 20") unbeweglich mit dem Schaft (16) verbunden ist, und
das feststehende und das angetriebene Arbeitselement (18, 20; 18', 20'; 18", 20") zwischen sich einen Arbeitsraum (F) bilden, der sich in proximaler Richtung verjüngt, und der zur Aufnahme von Körpergewebe dient, um dieses zu koagulieren und/oder zu schneiden.

2. Instrument nach Anspruch 1,
**dadurch gekennzeichnet, dass**
das feststehende Arbeitselement (20, 20', 20") Teil einer Schutzhülse (24) ist.

3. Instrument nach Anspruch 1,
**dadurch gekennzeichnet, dass**
das bewegliche Arbeitselement ( 18, 18', 18") aus einer in dem feststehenden Arbeitselement (20, 20', 20") vorgesehenen Stirnöffnung austritt.

4. Instrument nach Anspruch 1,
**dadurch gekennzeichnet, dass**
das feststehende Arbeitselement (20, 20', 20") eine zur Axialrichtung schräg verlaufende Stirnfläche (28, 28', 28") aufweist und/oder eine Stirnfläche (28, 28") aufweist, die insgesamt eben ausgebildet ist.

5. Instrument nach Anspruch 1,
**dadurch gekennzeichnet, dass**
das feststehende Arbeitselement (20, 20', 20") und das bewegliche Arbeitselement (18, 18', 18") in axialer Richtung am distalen Ende im wesentlichen gleich lang sind.

6. Instrument nach Anspruch 1,
**dadurch gekennzeichnet, dass**
das feststehende Arbeitselement (20') eine Stirnfläche (28) aufweist, die zumindest teilweise gekrümmt ausgebildet ist und/oder eine Stirnfläche (28) aufweist, die zur Längsachse des Instrumentes verschieden stark geneigte und/oder gekrümmte Abschnitte (a, b, c) aufweist.

7. Instrument nach Anspruch 1,
**dadurch gekennzeichnet, dass**
das feststehende Arbeitselement (20, 20', 20") verglichen zu dem Schaft (24) seitlich verjüngt ist.

8. Instrument nach Anspruch 1,
**dadurch gekennzeichnet, dass**
das bewegliche Arbeitselement (18, 18') rotationssymmetrisch ausgebildet ist.

9. Instrument nach Anspruch 1,
**dadurch gekennzeichnet, dass**
das bewegliche Arbeitselement (18) sich in distaler Richtung konisch verjüngt.

10. Instrument nach Anspruch 1,
**dadurch gekennzeichnet, dass**
ein Arbeitselement (18") relativ zu dem anderen Arbeitselement (20") um seine Längsachse verstellbar ist und mindestens zwei unterschiedlich gestaltete Arbeitsflächen aufweist und/oder dass das bewegliche Arbeitselement (18, 18', 18") und das feststehende Arbeitselement (20, 20', 20") lösbar miteinander verbunden sind.
